(19) Europäisches Patentamt
European Patent Office
Office européen des brevets

(11) Numéro de publication : **0 133 103**
**B1**

(12) # FASCICULE DE BREVET EUROPÉEN

(45) Date de publication du fascicule du brevet : **11.11.87**

(51) Int. Cl.⁴ : **C 07 H 15/20**, A 61 K 31/70

(21) Numéro de dépôt : **84401496.9**

(22) Date de dépôt : **17.07.84**

(54) **Benzyl-phényl-osides, procédé de préparation et utilisation.**

(30) Priorité : **20.07.83 FR 8311982**

(43) Date de publication de la demande :
**13.02.85 Bulletin 85/07**

(45) Mention de la délivrance du brevet :
**11.11.87 Bulletin 87/46**

(84) Etats contractants désignés :
**AT BE CH DE FR GB IT LI LU NL SE**

(56) Documents cités :
**EP-A- 0 051 023**
**CHEMICAL ABSTRACTS, vol. 82, no. 15, 14 avril 1975, page 468, no. 98327c, Columbus, Ohio, USA; A. LEVAI et al.: "Circular dichroism. LXVI. Chiroptical properties of some mono- and polysubstituted phenyl glycosides"**
**CHEMICAL ABSTRACTS, vol. 95, no. 9, 31 août 1981, page 831, no. 81409b, Columbus, Ohio, USA; H. KAEMMERER et al.: "Attempts to react methylenediphenols with glucose derivatives and to condense O-phenylglucoside derivatives"**
**CHEMICAL ABSTRACTS, vol. 90, no. 5, 29 janvier 1979, page 8, no. 33707x, Columbus, Ohio, USA; P.A.F. DIXON et al.: "The fate of dichlorophen in the rat"**
**CHEMICAL ABSTRACTS, vol. 86, no. 7, 14 février 1977, page 21, no. 37568t, Columbus, Ohio, USA; A.J. GANDOLFI et al.: "Metabolism of hexachlorophene in the rabbit. Excretion, tissue distribution, and characterization of the urinary glucuronide conjugate"**

(73) Titulaire : **SOCIETE DE RECHERCHES INDUSTRIELLES S.O.R.I.**
**38, avenue Hoche**
**F-75008 Paris (FR)**

(72) Inventeur : **Samereth, Soth**
**2, Rue de la 2ème Escadre**
**F-21600 Longvic (FR)**
Inventeur : **Bellamy, François**
**Rue Basse Cidex 11**
**F-21910 Saulon La Rue (FR)**
Inventeur : **Chazan, Jean-Bernard**
**4 Quai Nicolas Rolin**
**F-21000 Dijon (FR)**

(74) Mandataire : **Combe, André et al**
**CABINET BEAU DE LOMENIE 55, rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 133 103 B1**

Jouve, 18, rue St-Denis, 75001 Paris, France

**Description**

La présente invention concerne, en tant que produits industriels nouveaux, les benzyl-phényl-osides de formule I ci-après et leurs sels. Elle concerne également le procédé de préparation de ces produits et les compostions thérapeutiques les contenant ainsi que les compositions thérapeutiques contenant à titre d'ingrédients actifs l'un des deux composés ci-après, à savoir :

le [4-méthyl 2-(2-hydroxy 5-méthylbenzyl) phényl] 2,3,4,6-tétra-O-acétyl-α-D-glycanopyranosyle et

le [4-méthyl 2-(2-hydroxy 5-méthylbenzyl) phényl] x,y-di-O-acétylglucopyranosyle.

Ces deux composés sont connus et décrits notamment par Kaemmerer et al. dans Makromol. Chem. 1981 182(5) 1351-61, cité dans le Chemical Abstracts vol. 95 n° 9, 31 Août 1981, n° 81409b.

On sait que, dans le passé, on a proposé d'utiliser des phényl-glycosides comme agents doués de propriétés antivirales, voir à cet effet l'article de HIROSHI ARITA, Carbohydrate Research 62, 143-154 (1978).

On sait également que l'on a déjà proposé dans EP-A-51 023 des dérivés de benzoyl- et α-hydroxybenzyl-phényl-osides utiles en tant qu'agents anti-ulcéreux, anti-agrégants plaquettaires, antithrombotiques et oxygénateurs cérébraux.

On a trouvé de façon surprenante que, en remplaçant les groupes CO et CHOH situés entre les deux noyaux phényle de la formule générale de EP-A-51023 par un groupe $CH_2$, on obtient des composés particulièrement intéressants en tant qu'agents hypocholestérolémiants et hypolipidémiants, alors que les dérivés benzoyl-phényl-osides et α-hydroxybenzyl-phényl-osides antérieurement connus sont dénués d'effets hypocholestérolémiant et hypolipidémiant.

Les nouveaux produits selon l'invention sont caractérisés en ce qu'ils sont choisis parmi l'ensemble constitué par

(i) les benzyl-phényl-osides de formule générale

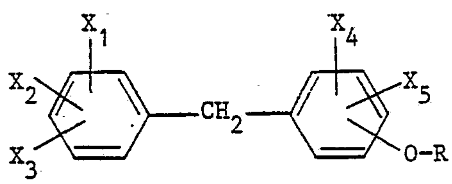

(I)

dans laquelle :

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle [$-SO_2CH_3$].

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées ; à l'exception des produits suivants :

[4-méthyl-2-(2-hydroxy 5-méthylbenzyl) phényl] 2,3,4,6-tétra-O-acétyl-α-D-glucanopyranosyl et

[4-méthyl 2-(2-hydroxy 5-méthylbenzyl) phényl] et x,y-di-O-acétylglucopyranosyl

(ii) leurs sels d'addition d'acide quand au moins un des groupes $X_1$, $X_2$, $X_3$, $X_4$, $X_5$ ou R comprend un reste basique.

Le groupe —O—R, compte tenu de la structure, de la formule I donnée ci-dessus, peut être en position ortho, méta ou para par rapport au groupe $CH_2$ situé entre les deux noyaux phényle.

Le terme « ose » qui intervient dans la définition du radical R désigne ici toute unité élémentaire glucidique non hydrolysable de formule brute $(CH_2O)n$, c'est-à-dire les carbohydrates monosaccharidiques non hydrolysables, d'une part, et l'α-L-rhamnose qui est un dérivé désoxyose de formule brute $C_6H_{12}O_5$, d'autre part. De plus, selon l'invention, l'atome d'hydrogène de chaque fonction hydroxyle du radical ose peut être remplacé par un groupe $COCH_3$, et la fonction hydroxyle de l'atome de carbone en position 2 peut être remplacée par une fonction amine, elle-même susceptible d'être substituée par un groupe $COCH_3$.

Par suite, R représente notamment un radical glycosyle tel que β-D-glucosyle, β-D-xylosyle, β-D-galactosyle, α-L-arabinosyle, β-D-glucosaminyle ou α-L-rhammosyle, les fonctions hydroxyle et amine pouvant, le cas échéant, être substituées par un groupe acétyle.

Par atome d'halogène, on entend ici les atomes de fluor, de chlore, de brome et d'iode, les halogènes préférés étant le fluor, le chlore et le brome, parmi ceux-ci les halogènes les plus intéressants du point de

vue thérapeutique sont le chlore et le brome.

Parmi les composés de formule I qui sont préférés selon l'invention, on peut notamment mentionner les dérivés où $X_1$, $X_2$, $X_3$, $X_4$ et $X_5$, qui peuvent être identiques ou différents, représentent chacun H, Cl, Br, $CH_3$, $CF_3$, OH, $OCH_3$, $NO_2$, $NH_2$, $N(CH_3)_2$, $SCH_3$, $SOCH_3$, $SO_2CH_3$, et R représente un radical choisi parmi l'ensemble constitué par les radicaux α-L-rhamnosyle, β-D-glucosyle, β-D-xylosyle, β-D-galactosyle, α-L-arabinosyle et β-D-glucosaminyle, dans lesquels, le cas échéant, les fonctions OH et $NH_2$ peuvent être acétylées.

Les composés de formule I peuvent être préparés selon une méthode connue en soi selon un mécanisme réactionnel classique. Le procédé que l'on préconise consiste à réduire un composé de formule

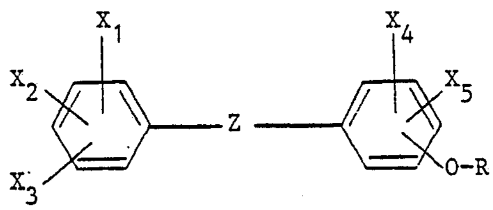

(II)

(où Z est CO ou CHOH ; $X_1$, $X_2$, $X_3$, $X_4$, $X_5$ et R sont définis comme indiqués ci-dessus) au moyen d'un agent réducteur choisi parmi $NaBH_4$ et $KBH_4$, dans l'acide trifluoracétique.

Le meilleur mode de mise en œuvre de ce procédé consiste successivement (i) à introduire l'agent réducteur dans le mélange comprenant le composé II et l'acide trifluoracétique, à une température inférieure ou égale à 0 °C, et de préférence comprise entre la température de solidification du milieu réactionnel et 0 °C, à raison d'un excès d'agent réducteur par rapport au composé II, et (ii) ladite addition terminée, on laisse réagir sous agitation pendant 0,5 à 12 h, à une température comprise entre 0 °C et 20 °C.

En pratique, l'introduction de l'agent réducteur est réalisée par petite fraction pendant au moins 0,5 h.

Selon le meilleur mode donné ci-dessus, quand Z est CHOH, on utilise au moins 3 moles d'agent réducteur pour 1 mole de composé II ; et, quand Z est CO, on utilise au moins 6 moles d'agent réducteur pour 1 mole de composé II.

De plus, il est avantageux, pour solubiliser le produit II de départ, que l'acide trifluoracétique soit associé à un solvant chloré, notamment le chlorure de méthylène, dans le rapport $CF_3COOH—CH_2Cl_2$ (1 : 2) v/v.

Pour obtenir un composé de formule I, où le reste R n'est pas acétylé, on peut avoir intérêt à réduire un composé de formule II où R est acétylé pour obtenir un composé I où R est acétylé qui est alors soumis à une réaction de désacétylation, car

1) le dérivé acétylé II est plus soluble dans le milieu réactionnel que le dérivé II non acétylé, et

2) chaque groupe $COCH_3$ fixé sur le reste R n'est pas affecté par la réaction de réduction au moyen de $NaBH_4$ ou $KBH_4$.

La désacétylation du groupe R est réalisée avantageusement par chauffage à reflux dans un alcool inférieur en $C_1$-$C_3$, en présence de l'alcoolate métallique correspondant. De préférence, l'alcool inférieur sera ici le méthanol et l'alcoolate métallique sera le méthanolate de sodium ou de magnésium.

Selon l'invention, on propose une composition thérapeutique caractérisée en ce qu'elle renferme, en association avec un excipient physiologiquement acceptable, au moins un composé choisi parmi l'ensemble constitué par :

(i) les benzyl-phényl-osides répondant à la formule générale

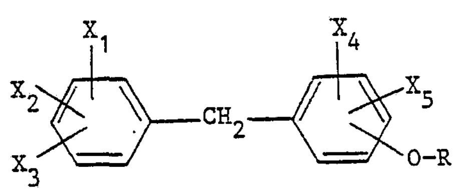

(I')

dans laquelle :

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R'' (où R' et R'', identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle,

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant suceptibles d'être acétylées ; et

(ii) leurs sels d'addition d'acide quand au moins un des groupes $X_1$, $X_2$, $X_3$, $X_4$, $X_5$ ou R comprend un reste basique.

Les composés de formule I' sont utiles en thérapeutique en tant qu'agents hypocholestérolémiants et hypolipidémiants. Ils sont utiles dans le traitement des hypercholestérolémies et des hyperlipidémies, et en particulier le traitement des surcharges lipidiques.

La propriété commune des composés de formule I' est constituée par les effets hypocholestérolémiant et hypolipidémiant ; à côté de cette propriété commune, certains composés, en particulier le produit de l'exemple 1 ci-après, présentent en outre des effets bénéfiques antithrombotiques.

D'autres caractéristiques et avantages de l'invention seront mieux compris à la lecture qui va suivre d'exemples de préparation nullement limitatifs mais donnés à titre d'illustration.

Préparation I

Obtention du [4-(4-nitrobenzyl)-phényl]-β-D-xylopyranoside (exemple 1 ; N° de code 646).

11 g (0,029 1 mole) de [4-(4-nitro-α-hydroxy-benzyl)-phényl]-β-D-xylopyranoside sont dissous dans 100 ml de $CH_2Cl_2$ anhydre et 50 ml d'acide trifluoroacétique sous courant d'azote. On additionne, à 0 °C, 3 g (0,077 3 mole) de tétraborohydrure de sodium par petites portions avec précaution. L'addition dure environ 45 min.

On continue à agiter à cette température jusqu'à ce que le produit de départ ait complètement disparu en contrôlant par C.C.M. [éluant : $CHCl_3$—$CH_3OH$ (1 : 1) v/v], soit environ 0,5 h. On verse alors le milieu réactionnel sur de la glace et on filtre le précipité formé. On lave ce précipité à l'eau froide jusqu'à pH neutre et on recristallise dans l'isopropanol. On recueille ainsi 10,2 g (rendement : 97 %) du produit attendu.

F = 166 °C

$[\alpha]_D^{20}$ = — 21° (c = 0,5 g/l ; méthanol)

Préparation II

Obtention du [4-(4-chlorobenzyl)-phényl]-2,3,4-tri-O-acétyl-β-D-xylopyranoside (exemple 44).

Dans un ballon de 250 ml muni d'une agitation énergique et sous courant d'azote, on introduit, à 0 °C, 7,1 g (0,014 mole) de [4-(4-chlorobenzoyl)-phényl]-2,3,4-tri-O-acétyl-β-D-xylopyranoside, puis 100 ml de chlorure de méthylène anhydre et 58 ml d'acide trifluoroacétique. En maintenant la température à 0 °C, on additionne par petites portions avec précaution 3,5 g (0,09 mole) de tétraborohydrure de sodium. La durée de cette addition est de l'ordre de 2 h. L'addition terminée, on agite à 0 °C pendant environ 3 h jusqu'à ce que le produit de départ ait complètement disparu en contrôlant en C.C.M. [éluant : $CH_3C_6H_5$—$CH_3CO_2C_2H_5$ (2 : 1) v/v]. On hydrolyse ensuite sur de la glace et on extrait plusieurs fois au chlorure de méthylène. On lave la phase organique ainsi obtenue à l'eau, puis avec une solution saturée de bicarbonate de sodium, et enfin à l'eau jusqu'à pH neutre. On sèche cette phase organique sur sulfate de sodium anhydre, on filtre puis on évapore sous pression réduite à 30-40 °C. Après cristallisation dans le méthanol, on obtient 6,5 g (rendement : 94 %) de produit attendu pur.

F = 101 °C

$[\alpha]_D^{20}$ = — 19° (c = 0,5 g/l ; acétate d'éthyle)

Préparation III

Obtention du [4-(4chlorobenzyl)-phényl]-β-D-xylopyranoside (exemple 9 ; N° de code 940).

On dissout 5,5 g (0,001 5 mole) de [4-(4-chlorobenzyl)-phényl]-2,3,4-tri-O-acétyl-β-D-xylopyranoside (produit de l'exemple 44 obtenu selon la préparation II ci-dessus) dans 150 ml de méthanol sous un courant d'azote à température ambiante (15-20 °C). On ajoute 1,8 ml de méthylate de sodium à environ 7 % p/v puis agite le milieu réactionnel à température ambiante pendant 2 h. On ajoute ensuite de la résine Amberlite IR 120 H®, en contrôlant le pH de la solution alcoolique. Lorsque le pH neutre est atteint, on filtre la résine et on évapore sous pression réduite à 30-40 °C et on recristallise le produit obtenu dans un mélange éthanol-eau (1 : 1) v/v. On obtient ainsi 2,5 g (rendement : 62 %) du produit attendu.

F = 149 °C

$[\alpha]_D^{20}$ = — 25° (c = 0,5 g/l ; $CH_3OH$)

Préparation IV

Obtention du [4-(3-nitrobenzyl)-phényl]-2,3,4-tri-O-acétyl-β-D-xylopyranoside (exemple 37)

Dans un ballon de 500 ml sous courant d'azote, on introduit successivement 17,3 g (0,034 5 mole) et [4-(3-nitrobenzoyl)-phényl]-2,3,4-tri-O-acétyl-β-D-xylopyranoside, 250 ml de chlorure de méthylène anhydre et 125 ml d'acide trifluoroacétique. On refroidit le milieu réactionnel à 0 °C et, sous agitation énergique, on ajoute 7,9 g (0,203 mole) de tétraborohydrure de sodium par petites fractions avec précaution. On agite ensuite pendant 2 h à 0 °C puis 1 h à température ambiante (15-20 °C). Le mélange réactionnel est ensuite hydrolysé sur de la glace. On extrait plusieurs fois avec du chlorure de méthylène, on lave à l'eau puis avec une solution saturée de $NaHCO_3$ et enfin à l'eau jusqu'à pH neutre. On sèche puis évapore sous pression réduite. Après recristallisation dans l'éther diisopropylique, on obtient 12,24 g (rendement : 73 %) du produit attendu.

F = 105 °C

$[\alpha]_D^{20}$ = — 25° (c = 0,5 g/l ; acétate d'éthyle).

Préparation V

Obtention du [4-(3-nitrobenzyl)-phényl]-β-D-xylopyranoside (exemple 2 ; N° de code 938).

12,24 g (0,025 mole) de [4-(3-nitrobenzyl)-phényl]-2,3,4-tri-O-acétyl-β-D-xylopyranoside (produit de l'exemple 37 obtenu selon la préparation IV) sont dissous à température ambiante (15-20 °C) sous agitation dans 200 ml de méthanol. On ajoute 3 ml de méthylate de sodium en solution à 7 % p/v. On agite à température ambiante jusqu'à totale disparition du produit de départ (soit environ 2 h). On ajoute ensuite de la résine Amberlite IR 120 H® jusqu'à pH neutre, puis on filtre. Le filtrat ainsi obtenu est évaporé sous pression réduite à 30-40 °C pour éliminer le méthanol ; on recristallise ensuite le résidu d'évaporation dans l'isopropanol et on obtient 6,93 g (rendement : 77 %) du produit attendu.

F = 139 °C

$[\alpha]_D^{20}$ = — 24,4° (c = 0,5 g/l ; MeOH)

Préparation VI

Obtention du [4-(benzyl)-phényl]-2,3,4-tri-O-acétyl-β-D-xylopyranoside (exemple 68).

Selon le mode opératoire décrit dans la préparation II, on obtient, à partir de 20 g de [4-(benzoyl)-phényl]-2,3,4-tri-O-acétyl-β-D-xylopyranoside, 13 g (rendement : 67 %) du produit attendu.

F = 112 °C

$[\alpha]_D^{20}$ = — 27° (c = 0,5 g/l ; acétate d'éthyle)

Préparation VII

Obtention du [4-(benzyl)-phényl]-β-D-xylopyranoside (exemple 33 ; N° de code 939).

Selon le mode opératoire décrit dans la préparation III, on obtient, à partir de 12 g de [4-(benzyl)-phényl]-2,3,4-tri-O-acétyl-β-D-xylopyranoside, 3,13 g du produit attendu.

F = 160 °C

$[\alpha]_D^{20}$ = — 28° (c = 0,5 g/l ; MeOH)

Préparation VIII

Obtention du [4-(4-méthylthiobenzyl) phényl]-β-D-xylopyranoside (exemple 85, N° de code 887)

Obtention du [4-(4-méthylthiobenzoyl)-phényl]-2,3,4-tri-O-acétyl-β-D-xylopyranoside.

18 g (0,073 6 mole) de (4-hydroxyphényl) (4-méthylthiophényl) méthanone sont dissous dans 500 $cm^3$ d'acétonitrile. A l'abri de la lumière, introduire 21 g (0,088 mole) d'oxyde d'argent et laisser sous agitation 15 min puis additionner 30 g (0,088 mole) d'acétobromoxylose. Laisser sous agitation pendant 3 h. Le milieu réactionnel est ensuite filtré et extrait au chlorure de méthylène. La phase organique obtenue est lavée à la soude 1N puis à l'eau jusqu'à pH neutre, puis séchée sur sulfate de magnésium et évaporée. Le résidu d'évaporation est cristallisé par addition d'éther.

On obtient 22,3 g du produit attendu (rendement 60,16 %)

F = 132 °C

Obtention du [4-(4-méthylthiobenzyl)-phényl]-2,3,4-tri-O-acétyl-β-D-xolopyranoside (exemple 86).

A partir du [4-(4-méthylthiobenzoyl) phényl]-2,3,4-tri-O-acétyl-β-D-xylopyranoside, selon le mode opératoire décrit dans la préparation II, on obtient le produit de l'exemple 86 sous forme d'une huile.

Obtention du produit de l'exemple 85.

Selon le mode opératoire décrit dans la préparation II, on obtient à partir de 6 g (0,012 mole) de [4-(4-méthylthiobenzoyl)-phényl]-2,3,4-tri-O-acétyl-β-D-xylopyranoside (produit de l'exemple 86) 3,9 g (rendement 86 %) du produit attendu après recristallisation dans un mélange éthanol-eau.
F = 140 °C
$[\alpha]_D^{20}$ = — 26 (C = 0,5 g/l ; méthanol).

Préparation IX

Obtention du [4-(4-méthylsulfinylbenzyl)-phényl]-β-D-xylopyranoside (exemple 87 ; N° de code 909).

On introduit 14,6 g (0,040 3 mole) de [4-(4-méthylthiobenzyl)-phényl]-β-D-xylopyranoside (produit de l'exemple 85 ; N° de code 887) dans 400 ml de méthanol.
A la solution obtenue, on ajoute ensuite 6,94 g (0,040 3 mole) d'acide m-chloroperoxy-benzoïque (MCPBA) et on laisse à température ambiante sous agitation pendant 12 h. On contrôle la disparition totale du produit de départ par CCM (éluant : toluène/méthanol 3/1). Le milieu réactionnel est évaporé. L'acide est extrait à l'éther, le produit précipite. Le précipité obtenu est filtré et lavé à l'éther pour éliminer toutes traces de MCPBA. La phase éthérée est lavée à l'eau pour récupérer la totalité du produit attendu. La phase aqueuse ainsi obtenue et les cristaux sont réunis. On obtient un sirop que l'on cristallise dans un mélange alcool isopropylique/éther, puis les cristaux obtenus sont recristallisés dans l'alcool isopropylique.
On obtient 7,8 g du produit attendu (rendement 51 %).
F = 163 °C
$[\alpha]_D^{20}$ = (C = 0,5 g/l ; méthanol).

Préparation X

Obtention du [4-(4-mésylbenzyl)-phényl]-2,3,4,5 tétra-O-acétyl-β-D-glucopyranoside (exemple 92)

Selon le mode opératoire décrit dans la préparation II à partir de 11,1 g (0,018 3 mole) de [4-(4-mésylbenzoyl)-phényl]-2,3,4,5-tétra-O-acétyl-β-D-glucopyranoside, on obtient 10,44 g du produit attendu sous forme d'un solide amorphe (rendement : 96 %).

Préparation XI

Obtention du [4-(4-méthylbenzyl)-phényl]-β-D-glucopyranoside (exemple 91 ; N° de code 1059)

Selon le mode opératoire décrit dans la préparation III, on obtient à partir de 8,1 g (0,013 6 mole) de [4-(4-mésylbenzyl)-phényl]-tétra-O-acétyl-β-D-glucopyranoside 4,9 g du produit attendu sous forme d'une poudre jaune amorphe obtenue par lyophilisation (rendement : 84 %).
$[\alpha]_D^{20}$ = — 41 (C = 0,5 ; $CH_3OH$).

Préparation XII

Obtention du [4-(4-mésylbenzyl)-phényl]-3,4,5-tri-O-acétyl-β-D-N-acétylglucosaminopyranoside) (exemple 94)

Selon le mode opératoire décrit dans la préparation II à partir de 5 g (0,008 6 mole) de [4-(4-mésylbenzoyl)-phényl]-2,3,4-tri-O-acétyl-β-D-N-acétylglucosaminopyranoside, on obtient 4 g du produit attendu sous forme d'un solide amorphe (rendement : 83 %).

Préparation XIII

Obtention du [4-(4-mésylbenzyl)-phényl]-β-D-N-acétylglucosaminopyranoside) (exemple 93 ; N° de code 1088).
Selon le mode opératoire décrit dans la préparation III, à partir de 3,9 g (0,006 9 mole) de [4-(4-mésylbenzyl)-phényl]-3,4,5-tri-O-acétyl-β-D-N-acétylglucosaminopyranoside, on obtient 2,21 g du produit attendu (rendement : 73,6 %).
F = 156 °C
$[\alpha]_D^{20}$ = + 7 (C = 0,5 ; $CH_3OH$).

Préparation XIV

Obtention du [4-(4-mésylbenzoyl)-phényl]-2,3,4-tri-O-acétyl-β-D-xylopyranoside) (exemple 90)

Obtention de la (4-hydroxy-phényl) (4-mésylphényl)-méthanone.

25 g (0,102 mole) de (4-hydroxyphényl) (4-méthylthiophényl) méthanone sont dissous dans 500 ml de méthanol.

On ajoute 58 g (0,34 mole) d'acide métachloroperoxybenzoïque (MCPBA). Le milieu réactionnel est ensuite porté à 40 °C et est laissé sous agitation pendant 48 h. Le méthanol est ensuite évaporé, le milieu réactionnel est repris par de l'acétate d'éthyle.

Cette phase organique est lavée à l'eau jusqu'à pH neutre, puis séchée sur sulfate de magnésium, puis évaporée.

On obtient 26,1 g de solide jaune (rendement : 93 %).

Obtention du [4-(4-mésylbenzoyl)-phényl]-2,3,4-tri-O-acétyl-β-D-xylopyranoside.

Sous atmosphère d'azote, à l'abri de la lumière, 26 g (0,094 mole) de (4-hydroxyphényl-(4-mésylphényl)-méthanone sont dissous partiellement dans 600 ml de chlorure de méthylène anhydre. On ajoute ensuite successivement 47,3 g (0,14 mole) d'acétobromoxylose, 76,5 g (0,56 mole) de $ZnCl_2$ et 19,8 g (0,11 mole) d'imidazolate d'argent.

On obtient une solution jaune que l'on chauffe à 40 °C sous agitation mécanique pendant 12 h. le milieu réactionnel est ensuite filtré, le filtrant est lavé avec 1 l de chlorure de méthylène. La phase organique est ensuite lavée avec de l'acide chlorhydrique 1N puis à l'eau, puis à la soude à 4 % et à nouveau à l'eau jusqu'à pH neutre. Après séchage sur sulfate de magnésium, le chlorure de méthylène est évaporé et on obtient 49,9 g de sirop jaune visqueux (rendement : 98 %).

Obtention du [4-(mésylbenzyl)-phényl]-2,3,4-tri-O-acétyl-β-D-xylopyranoside (exemple 90).

Dans un ballon de 2 l sous courant d'azote, on introduit successivement 49,9 g (0,094 mole) de [4-(4-mésylbenzoyl)-phényl]-2,3,4-tri-O-acétyl-β-D-xylopyranoside, 500 ml de chlorure de méthylène anhydre et 250 ml d'acide trifluoroacétique.

Le milieu réactionnel passe d'une coloration jaune à une coloration orangée.

Le milieu réactionnel est refroidi et maintenu à 0 °C sous agitation et on ajoute 18 g (0,47 mole) de tétraborohydrure de sodium par petites fractions. On agite ensuite en laissant remonter progressivement la température pendant 12 h. Le mélange réactionnel est ensuite hydrolysé sur glace, extrait plusieurs fois au chlorure de méthylène, lavé à l'eau puis avec une solution de bicarbonate de sodium et enfin à l'eau jusqu'à pH neutre. La phase organique obtenue est séchée sur sulfate de magnésium, puis évaporée sous pression réduite. on obtient 40,4 g du produit attendu (rendement : 83 %).

Préparation XV

Obtention du [4-(4-mésylbenzyl)-phényl]-β-D-xylopyranoside (exemple 89 ; N° de code 1008).

20 g (0,038 mole) de [4-(4-mésylbenzyl)-phényl]-2,3,4-tri-O-acétyl-β-D-xylopyranoside sont dissous à température ambiante dans 350 ml de méthanol. On ajoute 5,7 ml de méthylate de sodium en solution à 7 % p/v. On agite à température ambiante jusqu'à disparition complète du produit de départ (1 h 30). On ajoute ensuite de la résine Amberlite IR 120 H® jusqu'à pH neutre, puis on filtre. Le filtrat obtenu est évaporé sous pression réduite à 40 °C, on obtient ainsi 14,6 g de sirop qui cristallise par addition d'eau. Les cristaux sont filtrés et le produit obtenu est ensuite recristallisé dans un mélange alcool isopropylique-éther isopropylique.

On obtient 8,73 g du produit attendu (rendement : 58 %).

$[\alpha]_D^{19} = -10$ (C = 0,5 ; MeOH).

De façon non limitative, on a consigné dans le tableau I ci-après (où la position des substituants a été donnée arbitrairement, la numérotation des sommets des noyaux phényle étant effectuée à partir du groupe $CH_2$ central) un certain nombre de composés de formule I selon l'invention.

On a résumé dans le tableau II ci-après les caractéristiques physiques des composés selon l'invention et, dans le tableau III ci-après, les résultats des essais (toxicité, activité hypocholestérolémiante) entrepris sur un certain nombre de produits selon l'invention. Les protocoles opératoires qui ont été utilisés son les suivants.

Toxicité aiguë

La toxicité aiguë a été étudiée chez la souris par voie I.P. Elle est exprimée sous forme de DL-50 (dose létale entraînant la mort de la moitié des animaux) ou de DL-O (dose maximale non létale).

Activité hypocholestérolémiante

L'activité hypocholestérolémiante a été étudiée chez le rat mâle Wistar (pesant environ 200 à 220 g). Des lots de 10 rats par produit et par dose sont mis à jeun la veille de l'expérimentation. Les produits à tester sont administrés dans une solution d'eau gommeuse (gomme arabique à 30 g/l) aux instants T = 0 et T = + 7 h. Le taux de cholestérol dans le plasma est mesuré aux instants T = 0 et T = + 24 h, puis on calcule le pourcentage de variation entre T = 0 et T = + 24 h pour les lots traités et les lots témoins. Les résultats sont consignés dans le tableau III.

Les produits de formule (I) peuvent être administrés notamment par voie orale, sous forme de gélules ou de comprimés dragéifiés ou non renfermant chacun 0,05 à 1,5 g d'au moins un composé de formule (I) en tant que principe actif, et de préférence 0,1 à 0,9 g d'une part, et par voie injectable, sous forme de solutions renfermant de 0,05 à 0,5 g de principe actif dans 2 à 10 $cm^3$ d'eau distillée, d'autre part. Ces formes galéniques pourront être administrées à raison de 1 à 4 prises par jour.

(Voir tableaux pages 9 à 20)

Tableau I

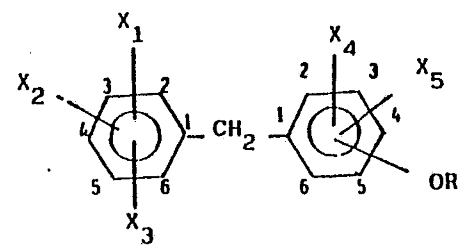

| Exemple | N° de code | $X_1$ | $X_2$ | $X_3$ | $X_4$ | $X_5$ | Position OR | R |
|---|---|---|---|---|---|---|---|---|
| 1 | 646 | $4-NO_2$ | H | H | H | H | para | β-D-xyl |
| 2 | 938 | $3-NO_2$ | H | H | H | H | para | β-D-xyl |
| 3 | 1090 | $4-NO_2$ | H | H | H | H | para | β-D-glu-NHAc |
| 4 | 1311 | $4-NO_2$ | H | H | H | H | para | β-D-Gal |
| 5 | 1346 | $4-NO_2$ | H | H | H | H | para | α-L-Rham |
| 6 | 1244 | $4-NO_2$ | H | H | H | H | para | α-L-Arab |
| 7 | 1089 | 2-Cl | H | H | H | H | para | β-D-xyl |
| 8 | 1208 | 3-Cl | H | H | H | H | para | β-D-xyl |
| 9 | 940 | 4-Cl | H | H | H | H | para | β-D-xyl |
| 10 | 1211 | 4-Br | H | H | H | H | para | β-D-xyl |

Tableau I (Suite)

| Exemple | N° de code | $X_1$ | $X_2$ | $X_3$ | $X_4$ | $X_5$ | Position OR | R |
|---|---|---|---|---|---|---|---|---|
| 11 | 1091 | 4-$CH_3$ | H | H | H | H | para | ß-D-xyl |
| 12 | 1092 | 2-$CH_3$ | H | H | H | H | para | ß-D-xyl |
| 13 | 1207 | 3-$CH_3$ | H | H | H | H | para | ß-D-xyl |
| 14 | | 4t-Bu | H | H | H | H | para | ß-D-xyl |
| 15 | 1133 | 4-$OCH_3$ | H | H | H | H | para | ß-D-xyl |
| 16 | | 2-$OCH_3$ | H | H | H | H | para | ß-D-xyl |
| 17 | 1135 | 3-$OCH_3$ | H | H | H | H | para | ß-D-xyl |
| 18 | 1305 | 4-$CF_3$ | H | H | H | H | para | ß-D-xyl |
| 19 | 1134 | 4-$NH_2$ | H | H | H | H | para | ß-D-xyl |
| 20 | 1352 | 4-$N(CH_3)_2$ | H | H | H | H | para | ß-D-xyl |
| 21 | | 4-NHipr | H | H | H | H | para | ß-D-xyl |
| 22 | | 2-$NH_2$ | H | H | H | H | para | ß-D-xyl |
| 23 | 1252 | 3-$NH_2$ | H | H | H | H | para | ß-D-xyl |
| 24 | | 4-$NO_2$ | H | H | H | H | méta | ß-D-xyl |

Tableau I (Suite)

| Exemple | N° de code | $X_1$ | $X_2$ | $X_3$ | $X_4$ | $X_5$ | Position OR | R |
|---|---|---|---|---|---|---|---|---|
| 25 | 1364 | $4-NO_2$ | H | H | H | H | ortho | ß-D-xyl |
| 26 | 1136 | 2-Cl | 4-Cl | H | H | H | para | ß-D-xyl |
| 27 | 1197 | $2-CH_3$ | $4-CH_3$ | $6-CH_3$ | H | H | para | ß-D-xyl |
| 28 | | $3-OCH_3$ | $4-OCH_3$ | $5-OCH_3$ | H | H | para | ß-D-xyl |
| 29 | 1206 | 4-OH | H | H | H | H | para | ß-D-xyl |
| 30 | 1209 | $4-CH_3$ | H | H | $3-CH_3$ | $5-CH_3$ | para | ß-D-xyl |
| 31 | | $4-NO_2$ | H | H | $3-OCH_3$ | $5-OCH_3$ | para | ß-D-xyl |
| 32 | | H | H | H | $2-CH_3$ | H | para | ß-D-xyl |
| 33 | 939 | H | H | H | H | H | para | ß-D-xyl |
| 34 | 1328 | H | H | H | $3-NO_2$ | H | para | ß-D-xyl |
| 35 | | $4-NO_2$ | H | H | 3-Cl | H | ortho | ß-D-xyl |
| 36 | | $4-NO_2$ | H | H | H | H | para | $(OAc)_3$-ß-D-xyl |
| 37 | | $3-NO_2$ | H | H | H | H | para | $(OAc)_3$-ß-D-xyl |
| 38 | | $4-NO_2$ | H | H | H | H | para | $(OAc)_3$-ß-Glu-NHAc |

Tableau I (Suite)

| Exemple | N° de code | $X_1$ | $X_2$ | $X_3$ | $X_4$ | $X_5$ | position OR | R |
|---|---|---|---|---|---|---|---|---|
| 39 | | $4-NO_2$ | H | H | H | H | para | $(OAc)_4$-ß-D-Gal |
| 40 | 1245 | $4-NO_2$ | H | H | H | H | para | $(OAc)_3$-α L-Rham |
| 41 | | $4-NO_2$ | H | H | H | H | para | $(OAc)_3$-α L-Arab |
| 42 | | 2-Cl | H | H | H | H | para | $(OAc)_3$-ß-D-xyl |
| 43 | | 3-Cl | H | H | H | H | para | $(OAc)_3$-ß-D-xyl |
| 44 | | 4-Cl | H | H | H | H | para | $(OAc)_3$-ß-D-xyl |
| 45 | | 4-Br | H | H | H | H | para | $(OAc)_3$-ß-D-xyl |
| 46 | | $4-CH_3$ | H | H | H | H | para | $(OAc)_3$-ß-D-xyl |
| 47 | | $2-CH_3$ | H | H | H | H | para | $(OAc)_3$-ß-D-xyl |
| 48 | | $3-CH_3$ | H | H | H | H | para | $(OAc)_3$-ß-D-xyl |
| 49 | | 4-tBu | H | H | H | H | para | $(OAc)_3$-ß-D-xyl |
| 50 | | $4-OCH_3$ | H | H | H | H | para | $(OAc)_3$-ß-D-xyl |
| 51 | | $2-OCH_3$ | H | H | H | H | para | $(OAc)_3$-ß-D-xyl |

Tableau I (Suite)

| Exemple | Nô de code | $X_1$ | $X_2$ | $X_3$ | $X_4$ | $X_5$ | Position OR | R |
|---|---|---|---|---|---|---|---|---|
| 52 | | 3-$OCH_3$ | H | H | H | H | para | $(OAc)_3$ß-D-xyl |
| 53 | | 4 -$CF_3$ | H | H | H | H | para | $(OAc)_3$-ß-D-xyl |
| 54 | | 4-$NH_2$ | H | H | H | H | para | $(OAc)_3$-ß-D-xyl |
| 55 | | 4-$N(CH_3)_2$ | H | H | H | H | para | $(OAc)_3$-ß-D-xyl |
| 56 | | 4-$NHCH(CH_3)_2$ | H | H | H | H | para | $(OAc)_3$-ß-D-xyl |
| 57 | | 2 -$NH_2$ | H | H | H | H | para | $(OAc)_3$-ß-D-xyl |
| 58 | | 3-$NH_2$ | H | H | H | H | para | $(OAc)_3$-ß-D-xyl |
| 59 | | 4-$NO_2$ | H | H | H | H | méta | $(OAc)_3$-ß-D-xyl |
| 60 | | 4-$NO_2$ | H | H | H | H | ortho | $(OAc)_3$-ß-D-xyl |
| 61 | | 2-Cl | 4-Cl | H | H | H | para | $(OAc)_3$-ß-D-xyl |
| 62 | 1199 | 2-$CH_3$ | 4-$CH_3$ | 6-$CH_3$ | H | H | para | $(OAc)_3$-ß-D-xyl |
| 63 | | 3-$OCH_3$ | 4-$OCH_3$ | 5-$OCH_3$ | H | H | para | $(OAc)_3$-ß-D-xyl |
| 64 | | 4-OH | H | H | H | H | para | $(OAc)_3$-ß-D-xyl |
| 65 | | 4-$NO_2$ | H | H | 3-$CH_3$ | 5-$CH_3$ | para | $(OAc)_3$-ß-D-xyl |

Tableau I (Suite)

| Exemple | N° de code | $X_1$ | $X_2$ | $X_3$ | $X_4$ | $X_5$ | Position OR | R |
|---|---|---|---|---|---|---|---|---|
| 66 | | 4-$OCH_3$ | H | H | 3-$OCH_3$ | 5-$OCH_3$ | para | $(OAc)_3$-ß-D-xyl |
| 67 | | H | H | H | 2-$CH_3$ | H | para | $(OAc)_3$-ß-D-Xyl |
| 68 | | H | H | H | H | H | para | $(OAc)_3$-ß-D-Xyl |
| 69 | | H | H | H | 3-$NO_2$ | H | para | $(OAc)_3$-ß-D-Xyl |
| 70 | | 4-$NO_2$ | H | H | 5-Cl | H | ortho | $(OAc)_3$-ß-D-xyl |
| 71 | 1205 | 4-$NO_2$ | H | H | H | H | para | ß - D - Glu |
| 72 | | 4-$NO_2$ | H | H | H | H | para | $(OAc)_4$-ß-D-Glu |
| 73 | | 4-$CH_3$ | H | H | 3-Cl | 5-Cl | para | ß-D-xyl |
| 74 | | 4-$CH_3$ | H | H | 3-Cl | 5-Cl | para | $(OAc)_3$-ß-D-xyl |
| 75 | 1354 | 4-Cl | H | H | H | H | méta | β-D-xyl |

Tableau I (Suite)

| Exemple | N° de code | $X_1$ | $X_2$ | $X_3$ | $X_4$ | $X_5$ | Position OH | R |
|---|---|---|---|---|---|---|---|---|
| 76 | | 4-Cl | H | H | H | H | méta | $(OAc)_3$β-Dxyl |
| 77 | 1353 | H | H | H | H | H | méta | β-D-xyl |
| 78 | | H | H | H | H | H | méta | $(OAc)_3$β-Dxyl |
| 79 | 1212 | H | H | H | 3-$CH_3$ | H | para | β-D-xyl |
| 80 | | H | H | H | 3-$CH_3$ | H | para | $(OAc)_3$β-Dxyl |
| 81 | | 4-$CH_3$ | H | H | 3-$CH_3$ | 5-$CH_3$ | para | $(OAc)_3$β-Dxyl |
| 82 | 1198 | 4-$NO_2$ | H | H | 3-$CH_3$ | 5-$CH_3$ | para | β-D-xyl |
| 83 | | 4-NHAc | H | H | H | H | méta | β-D-xyl |
| 84 | | 4-NHAc | H | H | H | H | méta | $(OAc)_3$β-Dxyl |
| 85 | 887 | 4-$SCH_3$ | H | H | H | H | para | β-D-xyl |
| 86 | | 4-$SCH_3$ | H | H | H | H | para | $(OAc)_3$β-Dxyl |
| 87 | 909 | 4-$SOCH_3$ | H | H | H | H | para | β-D-xyl |
| 88 | | 4-$SOCH_3$ | H | H | H | H | para | $(OAc)_3$-β-Dxyl |
| 89 | 1008 | 4-$SO_2CH_3$ | H | H | H | H | para | β-D-xyl |

Tableau I (Suite)

| Exemple | N° de code | $X_1$ | $X_2$ | $X_3$ | $X_4$ | $X_5$ | Position OH | R |
|---|---|---|---|---|---|---|---|---|
| 90 | | $4-SO_2CH_3$ | H | H | H | H | para | $(OAc)_3$β-D-xyl |
| 91 | 1059 | $4-SO_2CH_3$ | H | H | H | H | para | β- D- glu |
| 92 | | $4-SO_2CH_3$ | H | H | H | H | para | $(OAc)_4$β-Dglu |
| 93 | 1088 | $4-SO_2CH_3$ | H | H | H | H | para | β-DgluNHAc |
| 94 | | $4-SO_2CH_3$ | H | H | H | H | para | $(OAc)_3$β-DgluNHAc |

Tableau II

Constantes physiques

| Exemple | N° de code | F°C | $[\alpha]_D^{20}$ C : g/l |
|---|---|---|---|
| 1 | 646 | 166 | -21 C = 0,5 $CH_3OH$ |
| 2 | 938 | 139 | -24,4 C = 0,5 $CH_3OH$ |
| 3 | 1090 | 251 | -30 C = 0,5 DMF |
| 4 | 1311 | 162 | -29 C = 0,5 $CH_3OH$ |
| 5 | 1346 | 58 | -89 C = 0,5 $CH_3OH$ |
| 6 | 1244 | 99 | 0 C = 0,5 $CH_3OH$ |
| 7 | 1089 | 183 | -27 C = 0,5 $CH_3OH$ |
| 8 | 1208 | 153 | -24 C = 0,5 $CH_3OH$ |
| 9 | 940 | 149 | -25 C= 0,5 $CH_3OH$ |
| 10 | 1211 | 162 | -21 C = 0,5 $CH_3OH$ |
| 11 | 1091 | 165 | -28 C = 0,5 $CH_3OH$ |
| 12 | 1092 | 175 | -28 C = 0,5 $CH_3OH$ |
| 13 | 1207 | 154 | -26 C = 0,5 $CH_3OH$ |
| 15 | 1133 | 153 | -28 C = 0,5 $CH_3OH$ |
| 17 | 1135 | 138 | -29 C = 0,5 $CH_3OH$ |
| 18 | 1305 | 158 | -23 C = 0,5 $CH_3OH$ |
| 19 | 1134 | 166 | -30 C = 0,5 $CH_3OH$ |
| 20 | 1352 | 160 | -26 C = 0,5 $CH_3OH$ |
| 23 | 1252 | 188 | -27 C = 0,5 $CH_3OH$ |
| 25 | 1364 | 197 | -43 C = 0,5 $CH_3OH$ |
| 26 | 1136 | 158 | -26 C = 0,5 $CH_3OH$ |
| 27 | 1197 | 222 | -21 C = 0,5 $CH_3OH$ |

Tableau II (Suite)

| Exemple | N° de code | F°C | $[\alpha]_D^{20}$ C : g/l |
|---|---|---|---|
| 29 | 1206 | 175 | -27 C = 0,5 $CH_3OH$ |
| 30 | 1209 | 150 | +22 C = 0,5 $CH_3OH$ (*) |
| 33 | 939 | 160 | -28 C = 0,5 $CH_3OH$. |
| 34 | 1328 | composé amorphe | -43 C = 0,5 $CH_3OH$ |
| 36 | | huile | -40 C = 0,5 $CHCl_3$ |
| 37 | | 105 | -25 C =0,5 $CH_3COOC_2H_5$ |
| 38 | | 227 | - 7 C = 0,5 $CH_3COOC_2H_5$ |
| 39 | | 118 | + 5 C = 0,5 $CH_3COOC_2H_5$ |
| 40 | 1245 | 62 | - 64 C= 0,5 $CH_3COOC_2H_5$ |
| 41 | | 70 | + 22 C= 0,5 $CH_3OH$ |
| 42 | | 85 | - 21 C= 0,5 $CH_3COOC_2H_5$ |
| 43 | | 119 | - 19 C= 0,5 $CH_3COOC_2H_5$ |
| 44 | | 101 | - 19 C= 0,5 $CH_3COOC_2H_5$ |
| 45 | | 112 | - 17 C= 0,5 $CH_3COOC_2H_5$ |
| 46 | | 114 | - 22 C =0,5 $CH_3COOC_2H_5$ |
| 47 | | 108 | - 22 C =0,5 $CH_3COOC_2H_5$ |
| 48 | | 90 | - 23 C =0,5 $CH_3COOC_2H_5$ |
| 50 | | 80 | - 21 C =0,5 $CH_3COOC_2H_5$ |
| 52 | | 108 | - 5 C =0,5 $CH_3COOC_2H_5$ |
| 53 | | 82 | - 21 C =0,5 $CH_3COOC_2H_5$ |
| 55 | | 145 | - 21 C =0,5 $CH_3COOC_2H_5$ |
| 58 | | 69 | - 21 C =0,5 $CH_3COOC_2H_5$ |
| 60 | | huile | - 40 C = 0,5 $CH_3COOC_2H_5$ |

(*) mélange de diastéréisomères

Tableau II (Suite)

| exemple | N°de Code | F°C | $[\alpha]^{20}_{D}$ C : g/l |
|---|---|---|---|
| 61 | | 99 | - 17 C = 0,5 $CH_3COOC_2H_5$ |
| 62 | 1199 | 133 | - 16 C = 0,5 $CH_3COOC_2H_5$ |
| 64 | | 110 | - 21 C = 0,5 $CH_3COOC_2H_5$ |
| 65 | | 73 | + 9 C = 0,5 $CH_3COOC_2H_5$ (*) |
| 68 | | 112 | -27 C = 0,5 $CH_3COOC_2H_5$ |
| 69 | | 140 | - 46 C = 0,5 $CH_3COOC_2H_5$ |
| 71 | 1205 | (**) | - 39 C = 0,5 $CH_3OH$ |
| 72 | | 128 | - 17 C = 0,5 $CH_3COOC_2H_5$ |
| 75 | 1354 | 134 | -35 C = 0,5 $CH_3OH$ |
| 76 | | 99 | - 44 C = 0,5 $CH_3COOC_2H_5$ |
| 77 | 1353 | 141 | - 38 C = 0,5 $CH_3OH$ |
| 78 | | 110 | - 46 C = 0,5 $CH_3COOC_2H_5$ |
| 79 | 1212 | 126 | - 31 C = 0,5 $CH_3OH$ |
| 80 | | 87 | - 29 C = 0,5 $CH_3COOC_2H_5$ |
| 81 | | 63 | + 3 C = 0,5 $CH_3COOC_2H_5$ (*) |
| 82 | 1109 | 135 | + 25 C = 0,5 $CH_3OH$ (*) |
| 83 | | 171 | - 28 C = 0,5 $CH_3OH$ |
| 84 | | huile | - 26 C = 0,5 $CH_3OH$ |
| 85 | 887 | 140 | - 26 C = 0,5 $CH_3OH$ |
| 87 | 909 | 163 | - 20 C = 0,5 $CH_3OH$ |
| 89 | 1008 | 146 | - 10 C = 0,5 $CH_3OH$ |
| 91 | 1059 | composé amorphe | - 41 C = 0,5 $CH_3OH$ |
| 93 | 1088 | 156 | + 7. C = 0,5 $CH_3OH$ |

(*) mélange de diastéréisomères
(**) double point de fusion : 84 et 135

Tableau III

| Exemple (+) | N° de code | $DL_0$, $DL_{50}$ mg/kg i.p. | Variation du taux de de cholestérol en % | |
|---|---|---|---|---|
| | | | dose administrée p.o. (mg/kg) | Variation chez le sujet traité (*) % |
| 1 | 646 | $DL_{50} = 650$ | 65 | - 39 |
| 2 | 938 | $DL_0 > 800$ | 100 | - 27 |
| 3 | 1090 | $DL_0 > 800$ | 100 | - 42 |
| 4 | 1311 | $DL_0 \geq 900$ | 100 | - 33 |
| 5 | 1346 | | 100 | - 7 |
| 6 | 1244 | $DL_0 \geq 900$ | 100 | - 29 |
| 7 | 1089 | $DL_0 > 800$ | 100 | - 20 |
| 8 | 1208 | $DL_{50} \geq 800$ | 100 | - 37 |
| 9 | 940 | $DL_0 > 800$ | 100 | - 37 |
| 10 | 1211 | $DL_0 \geq 900$ | 100 | - 39 |
| 11 | 1091 | $DL_0 > 800$ | 100 | - 19 |
| 12 | 1092 | $DL_0 > 800$ | 100 | - 28 |
| 13 | 1207 | $DL_0 \geq 800$ | 100 | - 24 |
| 15 | 1133 | $DL_0 > 800$ | 100 | - 47 |
| 17 | 1135 | $DL_0 > 1000$ | 100 | - 50 |
| 18 | 1305 | $DL_{50} > 900$ | 100 | - 17 |
| 19 | 1134 | $DL_0 > 800$ | 100 | - 29 |
| 20 | 1352 | | 100 | - 22 |
| 23 | 1252 | $DL_0 \geq 900$ | 100 | - 28 |
| 26 | 1136 | $DL_{50} = 640$ | 100 | - 8 |
| 27 | 1197 | $DL_0 \geq 800$ | 100 | - 13 |
| 29 | 1206 | $DL_0 \geq 800$ | 100 | - 37 |
| 30 | 1209 | $DL_{50} = 630$ | 100 | - 12 |
| 33 | 939 | $DL_0 > 800$ | 100 | - 38 |
| 34 | 1328 | $DL_{50} = 250$ | 25 | - 15 |
| 62 | 1199 | $DL_0 > 900$ | 100 | - 18 |
| 71 | 1205 | $DL_0 \geq 900$ | 55 | - 21 |
| 75 | 1354 | | 100 | - 43 |
| 77 | 1353 | | 100 | - 25 |
| 79 | 1212 | $DL_{50} = 320$ | 30 | - 37 |
| 82 | 1198 | $DL_0 > 800$ | 100 | - 11 |
| 85 | 887 | $DL_0 > 800$ | 100 | - 23 |
| 89 | 1008 | $DL_{50} = 450$ | 45 | - 30 |
| 91 | 1059 | $DL_0 > 800$ | 100 | - 40 |
| 93 | 1088 | $DL_0 > 800$ | 100 | - 36 |

(*) non corrigée de la variation chez le groupe témoin.
(+) produits préférés du point de vue de l'activité hypocholestérolémiante.

**Revendications** (pour les Etats contractants : BE, CH, DE, FR, GB, IT, LI, LU, NL, SE)

1. Dérivé oside, caractérisé en ce qu'il est choisi parmi l'ensemble constitué par (i) les benzyl-phényl-osides répondant à la formule générale

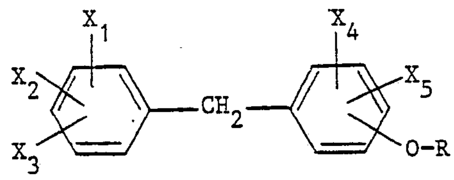

(I)

dans laquelle

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle,

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées à l'exception des produits suivants :

[4-méthyl 2-(2-hydroxy 5-méthylbenzyl) phényl] 2,3,4,6-tétra-O-acétyl-α-D-glucanopyranosyle et

[4-méthyl 2-(2-hydroxy 5-méthylbenzyl) phényl] x,y-di-O-acétylglucopyranosyle

(ii) leurs sels d'addition d'acide quand au moins un des groupes $X_1$, $X_2$, $X_3$, $X_4$, $X_5$ ou R comprend un reste basique.

2. Dérivé oside selon la revendication 1, caractérisé en ce que $X_1$, $X_2$, $X_3$, $X_4$ et $X_5$, qui peuvent être identiques ou différents, représentent chacun H, Cl, Br, $CH_3$, $CF_3$, OH, $OCH_3$, $NO_2$, $NH_2$, $N(CH_3)_2$, $SCH_3$, $SOCH_3$, $SO_2CH_3$, et R représente un radical choisi parmi l'ensemble constitué par les radicaux α-L-rhamnosyle, β-D-glucosyle, β-D-xylosyle, β-D-galactosyle, α-L-arabinosyle et β-D-glucosaminyle, dans lesquels, le cas échéant, les fonctions OH et NH peuvent être acétylées.

3. Dérivé oside selon la revendication 1, caractérisé en ce que R est choisi parmi l'ensemble constitué par les radicaux β-D-glucosyle, β-D-xylosyle, β-D-galactosyle, α-L-arabinosyle, β-D-glucosaminyle ou α-L-rhamnosyle, les fonctions hydroxyle et amine pouvant, le cas échéant, être substituées par un groupe acétyle.

4. [4-(4-Nitrobenzyl)-phényl]-β-D-xylopyranoside.

5. [4-(4-Chlorobenzyl)-phényl]-β-D-xylopyranoside.

6. [3-(4-chlorobenzyl)-phényl]-β-D-xylopyranoside.

7. [4-(4-mésylbenzyl)-phényl]-β-D-xylopyranoside.

8. Composition thérapeutique, caractérisée en ce qu'elle renferme, en association avec un excipient physiologiquement acceptable et au moins un composé choisi parmi l'ensemble constitué par

(i) les benzyl-phényl-osides de formule générale

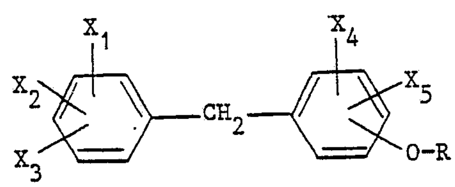

(I)

dans laquelle :

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle [$-SO_2CH_3$].

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées ; et

(ii) leurs sels d'addition d'acide quand au moins un des groupes $X_1$, $X_2$, $X_3$, $X_4$, $X_5$ ou R comprend un reste basique.

9. Procédé pour préparer un benzyl-phényl-oside de formule I selon la revendication 1, caractérisé en ce que l'on réduit un composé appartenant à la famille des benzyl- et α-hydroxybenzylphényl-osides de formule

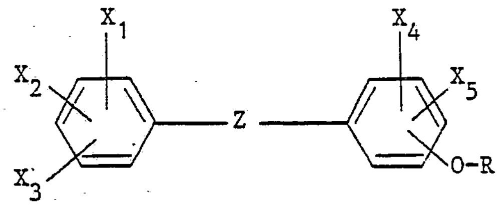

(II)

(où Z est CO ou CHOH ; $X_1$, $X_2$, $X_3$, $X_4$, $X_5$ et R sont définis comme indiqué ci-dessus) au moyen d'un agent réducteur choisi parmi $NaBH_4$ et $KBH_4$, dans l'acide trifluoroacétique.

10. Procédé selon la revendication 7, caractérisé en ce que, successivement

(i) on introduit dans le mélange compenant le composé II et l'acide trifluoroacétique l'agent réducteur à une température inférieure ou égale à 0 °C, pendant au moins 0,5 h, l'agent réducteur étant en excès par rapport au composé II ; et

(ii) ladite addition terminée, on laisse réagir sous agitation, à une température comprise entre 0 et 20 °C, pendant 0,5 à 12 h.

11. Utilisation d'une substance appartenant à l'ensemble constitué par :

(i) les benzyl-phényl-osides de formule générale

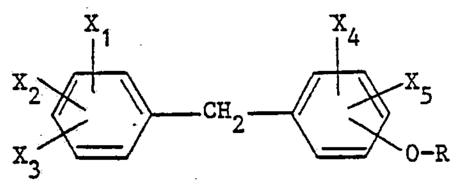

(I)

dans laquelle :

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle [$-SO_2CH_3$].

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées ; et

(ii) leurs sels d'addition d'acide quant au moins un des groupes $X_1$, $X_2$, $X_3$, $X_4$, $X_5$ ou R comprend un reste basique, pour la préparation d'une composition thérapeutique à action notamment hypocholestérolémiante ou hypolipidémiante.

12. Utilisation des composés définis dans la revendication 11 ci-dessus, pour la préparation d'un médicament hypocholestérolémiant et hypolipidémiant destiné à une utilisation thérapeutique vis-à-vis des surcharges lipidiques.

13. Utilisation du [4-(4-nitrobenzyl)-phényl)]-β-D-xylopyranoside pour la préparation d'un médicament anti-thrombotique.

**Revendications** (pour l'Etat contractant AT)

1. Procédé pour l'obtention des benzyl-phényl-osides répondant à la formule générale :

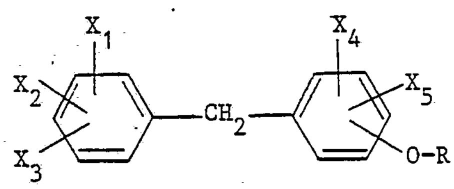

(I)

dans laquelle

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR'R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle,

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées à l'exception des produits suivants :

[4-méthyl 2-(2-hydroxy 5-méthylbenzyl) phényl] 2,3,4,6 tétra-O-acétyl-α-D-glucanopyranosyle et

[4-méthyl 2-(2-hydroxy 5-méthylbenzyl) phényl] x,y-di-O-acétylglucopyranosyle,

et de leurs sels d'addition d'acide quand au moins un des groupes $X_1$, $X_2$, $X_3$, $X_4$, $X_5$ ou R comprend un reste basique, caractérisé en ce que l'on réduit un composé appartenant à la famille des benzyl- et α-hydroxybenzyl-phényl-osides de formule

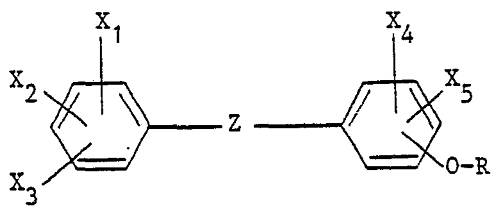

(II)

(où Z est CO ou CHOH ; $X_1$, $X_2$, $X_3$, $X_4$, $X_5$ et R sont définis comme indiqué ci-dessus) au moyen d'un agent réducteur choisi parmi $NaBH_4$ et $KBH_4$, dans l'acide trifluoroacétique, et en ce qu'éventuellement on transforme le composé ainsi obtenu en un sel d'addition d'acide quand au moins un des groupes $X_1$, $X_2$, $X_3$, $X_4$, $X_5$ ou R comprend un reste basique.

2. Procédé selon la revendication 1, caractérisé en ce que, successivement :

(i) on introduit dans le mélange comprenant le composé II et l'acide trifluoroacétique l'agent réducteur à une température inférieure ou égale à 0 °C, pendant au moins 0,5 h., l'agent réducteur étant en excès par rapport au composé II ; et

(ii) ladite addition terminée, on laisse réagir sous agitation, à une température comprise entre 0 et 20 °C, pendant 0,5 à 12 h.

3. Utilisation des dérivés osides choisis parmi l'ensemble constitué par :

(i) les benzyl-phényl-osides de formule générale

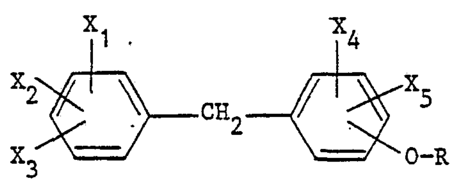

(I)

dans laquelle :

$X_1$, $X_2$, $X_3$, $X_4$ et $X_5$, identiques ou différents, représentent chacun un atome d'hydrogène, un atome d'halogène, un groupe alkyle en $C_1$-$C_4$, un groupe alkyle en $C_1$-$C_4$ substitué par un ou plusieurs atomes d'halogène (notamment un groupe $CF_3$), un groupe OH, un groupe alkoxy en $C_1$-$C_4$, un groupe alkoxy en $C_1$-$C_4$ substitué par un ou plusieurs atomes d'halogène, un groupe nitro, un groupe NR',R" (où R' et R", identiques ou différents, représentent chacun l'atome d'hydrogène, un groupe alkyle en $C_1$-$C_4$ ou un groupe acétyle), un groupe méthylthio, un groupe méthylsulfinyle ou un groupe mésyle [$-SO_2CH_3$].

R représente un radical ose choisi parmi l'ensemble constitué par

a) le radical α-L-rhamnosyle,

b) les radicaux monosaccharides non hydrolysables, et

c) les radicaux monosaccharides non hydrolysables où la fonction hydroxyle de l'atome de carbone en position 2 est remplacée par une fonction amine,

les fonctions hydroxyle et amine du groupe R étant susceptibles d'être acétylées ; et

(ii) leurs sels d'addition d'acide quant au moins un des groupes $X_1$, $X_2$, $X_3$, $X_4$, $X_5$ ou R comprend un reste basique, pour la préparation d'une composition thérapeutique à action notamment hypocholestérolémiante ou hypolipidémiante.

4. Utilisation des composés définis dans la revendication 3 ci-dessus, pour la préparation d'un

médicament hypocholestérolémiant et hypolipidémiant destiné à une utilisation thérapeutique vis-à-vis des surcharges lipidiques.

5. Utilisation du [4-(4-nitrobenzyl)-phényl)]-β-D-xylopyranoside pour la préparation d'un médicament anti-thrombotique.

**Claims** (for the Contracting States : BE, CH, DE, FR, GB, IT, LI, LU, NL, SE)

1. Oside derivative, characterized in that it is selected from the group constituted by

(i) the benzyl-phenyl-osides responding to the general formula

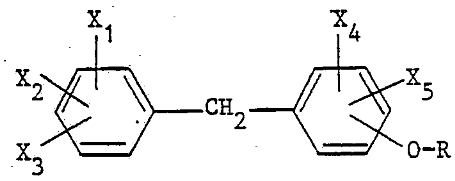

(I)

in which

$X_1$, $X_2$, $X_3$, $X_4$ and $X_5$, which are identical or different, each represent a hydrogen atom, a halogen atom, a $C_1$-$C_4$ alkyl group, a $C_1$-$C_4$ alkyl group substituted by one or more halogen atoms (in particular a $CF_3$ group), an OH group, a $C_1$-$C_4$ alkoxy group, a $C_1$-$C_4$ alkoxy group substituted by one or more halogen atoms, a nitro group, a group NR′R″ (in which R′ and R″, which are identical or different, each represent the hydrogen atom, a $C_1$-$C_4$ alkyl group or an acetyl group) a methylthio group, a methylsulfinyl group or a mesyl group, and

R represents an ose radical selected from the group comprising

a) the α-L-rhamnosyl radical,

b) non-hydrolyzable monosaccharide radicals, and

c) non-hydrolyzable monosaccharide radicals in which the hydroxyl group on the carbon atom in the 2-position is replaced with an amine group,

the hydroxyl and amine groups of the group R being capable of acetylation with the exception of the following products :

4-methyl 2-(2-hydroxy 5-methylbenzyl) phenyl 2,3,4,6-tetra-O-acetyl-α-D-glucanopyranosyl, and

4-methyl 2-(2-hydroxy 5-methylbenzyl) phenyl x,y-di-O-acetylglucopyranosyl,

(ii) their acid addition salts in case where at least one of the groups $X_1$, $X_2$, $X_3$, $X_4$, $X_5$ or R comprises a basic radical.

2. Oside derivative according to claim 1, characterized in that $X_1$, $X_2$, $X_3$, $X_4$ and $X_5$, which can be identical or different, each represent H, Cl, Br, $CH_3$, $CF_3$, OH, $OCH_3$, $NO_2$, $NH_2$, $N(CH_3)_2$, $SCH_3$, $SOCH_3$, $SO_2CH_3$, and R represents a radical selected from the group constituted by the α-L-rhamnosyl, β-D-glucosyl, β-D-xylosyl, β-D-galactosyl, α-L-arabinosyl and β-D-glucosaminyl radicals, in which, if appropriate, the OH and NH groups can be acylated.

3. Oside derivative according to claim 1, characterized in that R is selected from the group constituted by the β-D-glucosyl, β-D-xylosyl, β-D-galactosyl, α-L-arabinosyl, β-D-glucosaminyl or α-L-rhamnosyl radicals, it being possible, if appropriate, for the hydroxyl and amine groups to be substituted by an acetyl group.

4. 4-(4-Nitrobenzyl)phenyl beta-D-xylopyranoside.

5. 4-(4-Chlorobenzyl)phenyl beta-D-xylopyranoside.

6. 3-(4-Chlorobenzyl)phenyl beta-D-xylopyranoside.

7. 4-(4-Mesylbenzyl)phenyl beta-D-xylopyranoside.

8. Therapeutic composition, characterized in that it contains in association with a physiologically acceptable excipient and at least one compound selected from the group constituted by

(i) the benzyl-phenyl-osides of general formula

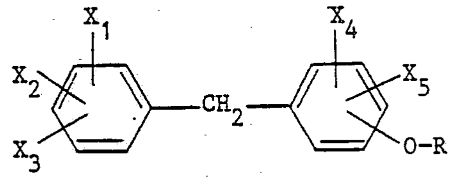

(I)

in which :

$X_1$, $X_2$, $X_3$, $X_4$ and $X_5$, identical or different, each represent a hydrogen atom, a halogen atom, a $C_1$-$C_4$ alkyl group, a $C_1$-$C_4$ alkyl group substituted by one or more halogen atoms (in particular a $CF_3$ group), an OH group, a $C_1$-$C_4$ alkoxy group, a $C_1$-$C_4$ alkoxy group substituted by one or more halogen atoms, a nitro group, a group NR′R″ (in which R′ and R″, which are identical or different, each represent the

hydrogen atom, a $C_1$-$C_4$ alkyl group or an acetyl group), a methylthio group, a methylsulfinyl group or a [$-SO_2CH_3$] mesyl group,

R represents an ose radical selected from the group comprising

a) the α-L-rhamnosyl radical,

b) non-hydrolyzable monosaccharide radicals, and

c) non-hydrolyzable monosaccharide radicals in which the hydroxyl group on the carbon atom in the 2-position is replaced with an amine group,

the hydroxyl and amine groups of the group R being capable of acetylation, and

(ii) their acid addition salts in case where at least one of the groups $X_1$, $X_2$, $X_3$, $X_4$, $X_5$ or R comprises a basic radical.

9. Method for the preparation of a benzyl-phenyloside of the formula I according to claim 1, characterized in that it comprises reducing a compound belonging to the family of the benzyl osides and α-hydroxybenzylphenyl osides of the formula

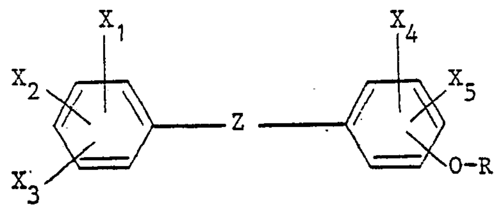

(II)

(in which Z is CO or CHOH and $X_1$, $X_2$, $X_3$, $X_4$, $X_5$ and R are defined as indicated above) by means of a reducing agent chosen from $NaBH_4$ and $KBH_4$, in trifluoroacetic acid.

10. Method according to claim 7, characterized in that it comprises successively

(i) introducing the reducing agent into a mixture comprising the compound of the formula II and the trifluoroacetic acid, at a temperature below or equal to 0 °C, over at least 0.5 hour, the reducing agent being in excess relative to the compound II ; and

(ii) when the said addition has ended, allowing the reaction to continue for 0.5 to 12 hours, at a temperature of between 0° and 20 °C, with stirring.

11. Use of a substance belonging to the group constituted by :

(i) the benzyl-phenyl-osides of general formula

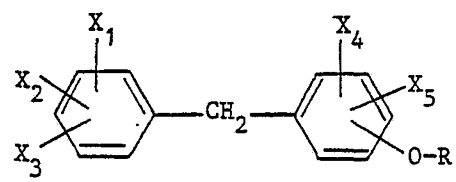

(I)

in which :

$X_1$, $X_2$, $X_3$, $X_4$ and $X_5$, identical or different, each represent a hydrogen atom, a halogen atom, a $C_1$-$C_4$ alkyl group, a $C_1$-$C_4$ alkyl group substituted by one or more halogen atoms (in particular a $CF_3$ group), an OH group, a $C_1$-$C_4$ alkoxy group, a $C_1$-$C_4$ alkoxy group substituted by one or more halogen atoms, a nitro group, a group NR'R" (in which R' and R", which are identical or different, each represent the hydrogen atom, a $C_1$-$C_4$ alkyl group or an acetyl group), a methylthio group, a methylsulfinyl group or a [$-SO_2CH_3$] mesyl group,

R represents an ose radical selected from the group comprising

-a) the α-L-rhamnosyl radical,

b) non-hydrolyzable monosaccharide radicals, and

c) non-hydrolyzable monosaccharide radicals in which the hydroxyl group on the carbon atom in the 2-position is replaced with an amine group,

the hydroxyl and amine groups of the group R being capable of acetylation, and

(ii) their acid addition salts in case where at least one of the groups $X_1$, $X_2$, $X_3$, $X_4$, $X_5$ or R comprises a basic radical, for the preparation of a therapeutic composition having in particular a hypocholesterolemic or hypolipidemic action.

12. Use of the compounds defined in claim 1, for the preparation of a hypocholesterolemic and hypolipidemic drug for a therapeutic treatment of lipid excesses.

13. Use of 4-(4-nitrobenzyl) phenyl β-D-xylopyranoside for the preparation of antithrombotic drug.

**Claims** (for the Contracting State AT)

1. Process for obtaining benzyl-phenyl-osides responding to the general formula :

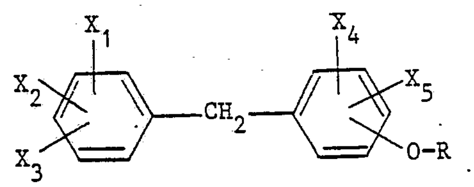

(I)

in which

$X_1$, $X_2$, $X_3$, $X_4$ and $X_5$, which are identical or different, each represent a hydrogen atom, a halogen atom, a $C_1$-$C_4$-alkyl group substituted by one or more halogen atoms (in particular a $CF_3$ group), an OH group, a $C_1$-$C_4$-alkoxy group, a $C_1$-$C_4$-alkoxy-group substituted by one ore more halogen atoms, a nitro group, a group NR'R" (in which R' and R", which are identical or different, each represent the hydrogen atom, a $C_1$-$C_4$-alkyl group or an acetyl group) a methylthio group, a methylsulfinyl group or a mesyl group, and

R represents an ose radical selected from the group comprising

a) the α-L-rhamnosyl radical,

b) non-hydrolyzable monosaccharide radicals, and

c) non-hydrolyzable monosaccharide radicals in which the hydroxyl group on the carbon atom in the 2-position is replaced with an amine group,

the hydroxyl and amine groups of the gorup R being capable of acetylation with the exception of the following products ;

4-methyl 2-(2-hydroxy 5-methylbenzyl) phenyl 2,3,4,6-tetra-O-acetyl-α-D-glucanopyranosyl, and

4-methyl 2-(2-hydroxy 5-methylbenzyl) phenyl x,y-di-O-acetylglucopyranosyl,

and their acid addition salts in case where at least one of the groups $X_1$, $X_2$, $X_3$, $X_4$, $X_5$ or R comprises a basic radical, characterized in that it comprises reducing a compound belonging to the family of the benzyl osides and α-hydroxybenzylphenyl osides of the formula

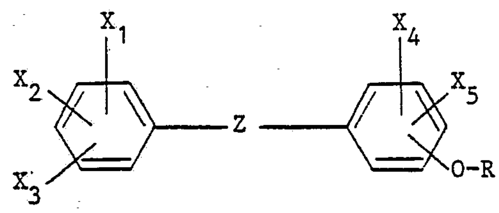

(II)

(in which Z is CO or CHOH ; $X_1$, $X_2$, $X_3$, $X_4$, $X_5$ and R are defined as indicated above) by means of a reducing agent chosen from $NaBH_4$ and $KBH_4$, in trifluoroacetic acid, and in that the compound thus obtained is optionally converted into an acid addition salt, in case where at least one of the groups $X_1$, $X_2$, $X_3$, $X_4$, $X_5$ or R comprises a basic radical.

2. Method according to claim 1, characterized in that it comprises successively

(i) introducing the reducing agent into a mixture comprising the compound of the formula II and the trifluoroacetic acid, at a temperature below or equal to 0 °C, over at least 0.5 hour, the reducing agent being in excess relative to the compound II ; and

(ii) when the said addition has ended, allowing the reaction to continue for 0.5 to 12 hours, at a temperature of between 0° and 20 °C, with stirring.

3. Use of oside derivatives belonging to the group constituted by :

(i) the benzyl-phenyl-osides of general formula

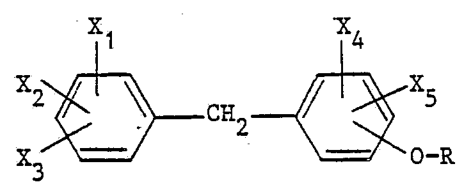

(I)

in which :

$X_1$, $X_2$, $X_3$, $X_4$ and $X_5$, identical or different, each represent a hydrogen atom, a halogen atom, a $C_1$-$C_4$-alkyl group, a $C_1$-$C_4$-alkyl group substituted by one or more halogen atoms (in particular a $CF_3$ group), an OH group, a $C_1$-$C_4$-alkoxy group, a $C_1$-$C_4$-alkoxy group substituted by one or more halogen atoms, a nitro group, a group NR'R" (in which R' and R", which are identical or different, each represent the hydrogen atom, a $C_1$-$C_4$-alkyl group or an acetyl group), a methylthio group, a methylsulfinyl group or a [$-SO_2CH_3$] mesyl group,

R represents an ose radical selected from the group comprising

a) the α-L-rhamnosyl radical,

b) non-hydrolyzable monosaccharide radicals, and

c) non-hydrolyzable monosaccharide radicals in which the hydroxyl group on the carbon atom in the 2-position is replaced with an amine group, the hydroxyl and amine groups of the group R being capable of acetylation, and

(ii) their acid addition salts in case where at least one of the groups $X_1$, $X_2$, $X_3$, $X_4$, $X_5$ or R comprises a basic radical, for the preparation of a therapeutic composition having in particular a hypocholesterolemic or hypolipidemic action.

4. Use of the compounds defined in above claim 3, for the preparation of a hypocholesterolemic and hypolipidemic drug for a therapeutic treatment of lipid excesses.

5. Use of 4-(4-nitrobenzyl) phenyl β-D-xylopyranoside for the preparation of an antithrombotic drug.

**Patentansprüche** (für die Vertragsstaaten : BE, CH, DE, FR, GB, IT, LI, LU NL, SE)

1. Osidderivat, dadurch gekennzeichnet, daß es ausgewählt ist aus der Gruppe bestehend aus

(i) den Benzyl-phenyl-osiden der allgemeinen Formel

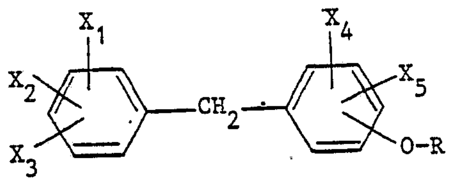

(I)

worin

$X_1$, $X_2$, $X_3$, $X_4$ und $X_5$, gleich oder verschieden, jeweils ein Wasserstoffatom, ein Halogenatom, eine $C_1$-$C_4$-Alkylgruppe, eine durch ein oder mehrere Halogenatome (insbesondere eine $CF_3$-Gruppe) substituierte $C_1$-$C_4$-Alkylgruppe, eine OH-Gruppe, eine $C_1$-$C_4$-Alkoxygruppe, eine durch ein oder mehrere Halogenatome substituierte $C_1$-$C_4$-Alkoxygruppe, eine Nitrogruppe, eine Gruppe NR'R'' (worin R' und R'', gleich oder verschieden, jeweils ein Wasserstoffatom, eine $C_1$-$C_4$-Alkylgruppe oder eine Acetylgruppe bedeuten), eine Methylthiogruppe, eine Methylsulfinylgruppe oder eine Mesylgruppe darstellen,

R einen Oserest darstellt, ausgewählt aus der Gruppe bestehend aus

a) α,L-Rhamnosylrest,

b) nicht hydrolysierbaren Monosaccharidresten, und

c) nicht hydrolysierbaren Monosaccharidresten, worin die Hydroxylfunktion des Kohlenstoffatoms in Position 2 durch eine Aminfunktion ersetzt ist,

wobei die Hydroxyl- und Aminfunktionen der Gruppe R acetyliert werden können, mit Ausnahme der folgenden Produkte :

[4-Methyl-2-(2-hydroxy-5-methylbenzyl)-phenyl]-2,3,4,6-tetra-O-acetyl-α-D-glucanopyranosyl und

[4-Methyl-2-(2-hydroxy-5-methylbenzyl)-phenyl]-x,y-di-O-acetylglucopyranosyl,

(ii) ihren Säureadditionssalzen, wenn mindestens eine der Gruppen, $X_1$, $X_2$, $X_3$, $X_4$, $X_5$ oder R einen basischen Rest umfaßt.

2. Osidderivat nach Anspruch 1, dadurch gekennzeichnet, daß $X_1$, $X_2$, $X_3$, $X_4$ und $X_5$, die gleich oder verschieden sein können jeweils für H, Cl, Br, $CH_3$, $CF_3$, OH, $OCH_3$, $NO_2$, $NH_2$, $N(CH_3)_2$, $SCH_3$, $SOCH_3$, $SO_2CH_3$ stehen und R einen Rest, ausgewählt aus der Gruppe bestehend aus den Resten α-L-Rhamnosyl, β-D-Glucosyl, β-D-Xylosyl, β-D-Galactosyl, α-L-Arabinosyl und β-D-Glucosaminyl bedeutet, in welchen Resten die OH— und NH— Funktionen gegebenenfalls acetyliert sein können.

3. Osidderivat nach Anspruch 1, dadurch gekennzeichnet, daß R ausgewählt ist aus der Gruppe bestehend aus den Resten β-D-Glucosyl, β-D-Xylosyl, β-D-Galactosyl, α-L-Arabinosyl, β-D-Glucosaminyl oder α-L-Rhamnosyl, wobei die Hydroxyl- und Aminfunktionen gegebenenfalls durch eine Acetylgruppe substituiert sein können.

4. [4-(4-Nitrobenzyl)-phenyl]-β-D-xylopyranosid.

5. [4-(4-Chlorbenzyl)-phenyl]-β-D-xylopyranosid.

6. [3-(4-Chlorbenzyl)-phenyl]-β-D-xylopyranosid.

7. [4-(4-Mesylbenzyl)-phenyl]-β-D-xylopyranosid.

8. Therapeutische Zusammensetzung, dadurch gekennzeichnet, daß sie in Verbindung mit einem physiologisch akzeptablen Exzipienten mindestens eine Verbindung enthält, welche ausgewählt ist aus der Gruppe bestehend aus

(i) den Benzyl-phenyl-osiden der allgemeinen Formel

(Siehe Formel Seite 28 f.)

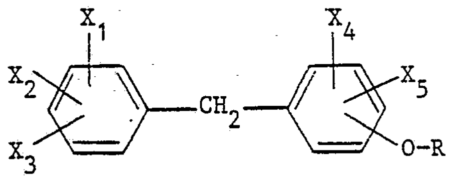

(I)

worin

$X_1$, $X_2$, $X_3$, $X_4$ und $X_5$, gleich oder verschieden, jeweils ein Wasserstoffatom, ein Halogenatom, eine $C_1$-$C_4$-Alkylgruppe, eine durch ein oder mehrere Halogenatome (insbesondere eine $CF_3$-Gruppe) substituierte $C_1$-$C_4$-Alkylgruppe, eine OH-Gruppe, eine $C_1$-$C_4$-Alkoxylgruppe, eine durch ein oder mehrere Halogenatome substituierte $C_1$-$C_4$-Alkoxygruppe, eine Nitrogruppe, eine Gruppe NR'R" (worin R' und R", gleich oder verschieden, jeweils ein Wasserstoffatom, eine $C_1$-$C_4$-Alkylgruppe oder eine Acetylgruppe bedeuten), eine Methylthiogruppe, eine Methylsulfinylgruppe oder eine Mesylgruppe [$-SO_2CH_3$] darstellen,

R einen Oserest darstellt, ausgewählt aus der Gruppe bestehend aus

a) α-L-Rhamnosylrest,

b) nicht hydrolysierbaren Monosaccharidresten, und

c) nicht hydrolysierbaren Monosaccharidresten, worin die Hydroxylfunktion des Kohlenstoffatoms in Position 2 durch eine Aminfunktion ersetzt ist,

wobei die Hydroxyl- und Aminfunktionen der Gruppe R acetyliert werden können ; und

(ii) ihren Säureadditionssalzen, wenn mindestens eine der Gruppen $X_1$, $X_2$, $X_3$, $X_4$, $X_5$ oder R einen basischen Rest umfaßt.

9. Verfahren zur Herstellung eines Benzyl-phenyl-osids der Formel I nach Anspruch 1, dadurch gekennzeichnet, daß man eine zur Familie der Benzyl- und α-Hydroxybenzylphenyl-oside der Formel

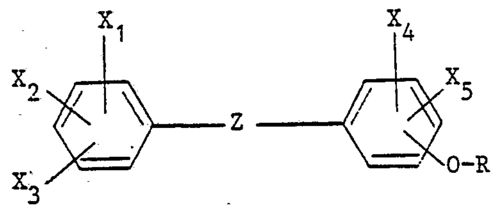

(II)

(worin Z für CO oder CHOH steht ; $X_1$, $X_2$, $X_3$, $X_4$, $X_5$ und R wie oben definiert sind) gehörende Verbindung mit einem Reduktionsmittel, ausgewählt aus $NaBH_4$ und $KBH_4$, in Trifluoressigsäure reduziert.

10. Verfahren nach Anspruch 9, dadurch gekennzeichnet, daß man nacheinander

(i) in eine die Verbindung II und Trifluoressigsäure enthaltende Mischung das Reduktionsmittel bei einer Temperatur von weniger oder gleich 0 °C während wenigstens 0,5 h einbringt, wobei das Reduktionsmittel, bezogen auf die Verbindung II, im Überschuß ist ; und

(ii) sobald die Zugabe beendet ist, unter Rühren bei einer Temperatur zwischen 0 und 20 °C während 0,5 bis 12 h reagieren läßt.

11. Verwendung einer Substanz aus der Gruppe bestehend aus :

(i) den Benzyl-phenyl-osiden der allgemeinen Formel

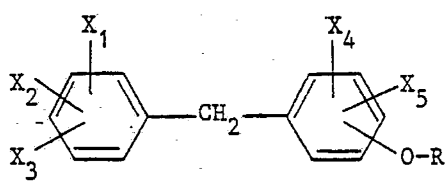

(I)

worin

$X_1$, $X_2$, $X_3$, $X_4$ und $X_5$, gleich oder verschieden, jeweils ein Wasserstoffatom, ein Halogenatom, eine $C_1$-$C_4$-Alkylgruppe, eine durch ein oder mehrere Halogenatome (insbesondere eine $CF_3$-Gruppe) substituierte $C_1$-$C_4$-Alkylgruppe, eine OH-Gruppe, eine $C_1$-$C_4$-Alkoxygruppe, eine durch ein oder mehrere Halogenatome substituierte $C_1$-$C_4$-Alkoxygruppe, eine Nitrogruppe, eine Gruppe NR'R" (worin R' und R", gleich oder verschieden, jeweils ein Wasserstoffatom, eine $C_1$-$C_4$-Alkylgruppe oder eine Acetylgruppe bedeuten), eine Methylthiogruppe, eine Methylsulfinylgruppe oder eine Mesylgruppe [$-SO_2CH_3$] darstellen,

R einen Oserest darstellt, ausgewählt aus der Gruppe bestehend aus

a) α-L-Rhamnosylrest,

b) nicht hydrolysierbaren Monosaccharidresten, und

c) nicht hydrolysierbaren Monosaccharidresten, worin die Hydroxylfunktion des Kohlenstoffatoms in Position 2 durch eine Aminfunktion ersetzt ist,

wobei die Hydroxyl- und Aminfunktionen der Gruppe R acetyliert werden können ; und

(ii) ihren Säureadditionssalzen, wenn mindestens eine der Gruppen $X_1$, $X_2$, $X_3$, $X_4$, $X_5$ oder R einen basischen Rest umfaßt, zur Herstellung einer therapeutischen Zusammensetzung mit insbesondere hypocholesterinämischer oder hypolipidämischer Wirkung.

12. Verwendung der im obigen Anspruch 11 definierten Verbindungen zur Herstellung eines hypocholesterinämischen und hypolipidämischen Medikaments zur therapeutischen Verwendung bei vermehrtem Lipidgehalt.

13. Verwendung von [4-(4-Nitrobenzyl)-phenyl]-β-D-xylopyranosid zur Herstellung eines antithrombotischen Medikaments.

**Patentansprüche** (für den Vertragsstaat AT)

1. Verfahren zur Herstellung von Benzyl-phenyl-osiden der allgemeinen Formel

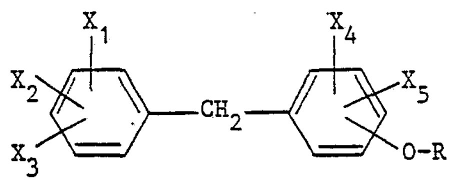

(I)

worin

$X_1$, $X_2$, $X_3$, $X_4$ und $X_5$, gleich oder verschieden, jeweils ein Wasserstoffatom, ein Halogenatom, eine $C_1$-$C_4$-Alkylgruppe, eine durch ein oder mehrere Halogenatome (insbesondere eine $CF_3$-Gruppe) substituierte $C_1$-$C_4$-Alkylgruppe, eine OH-Gruppe, eine $C_1$-$C_4$-Alkoxygruppe, eine durch ein oder mehrere Halogenatome substituierte $C_1$-$C_4$-Alkoxygruppe, eine Nitrogruppe, eine Gruppe NR'R'' (worin R' und R'', gleich oder verschieden, jeweils ein Wasserstoffatom, eine $C_1$-$C_4$-Alkylgruppe oder eine Acetylgruppe bedeuten), eine Methylthiogruppe, eine Methylsulfinylgruppe oder eine Mesylgruppe darstellen,

R einen Oserest darstellt, ausgewählt aus der Gruppe bestehend aus

a) α-L-Rhamnosylrest,

b) nicht hydrolysierbaren Monosaccharidresten, und

c) nicht hydrolysierbaren Monosaccharidresten, worin die Hydroxylfunktion des Kohlenstoffatoms in Position 2 durch eine Aminfunktion ersetzt ist,

wobei die Hydroxyl- und Aminfunktionen der Gruppe R acetyliert werden können, mit Ausnahme der folgenden Produkte :

[4-Methyl-2-(2-hydroxy-5-methylbenzyl)-phenyl]-2,3,4,6-tetra-O-acetyl-α-D-glucanopyranosyl und

[4-Methyl-2-(2-hydroxy-5-methylbenzyl)-phenyl]-x,y-di-O-acetylglucopyranosyl,

und ihren Säureadditionssalzen, wenn mindestens eine der Gruppen $X_1$, $X_2$, $X_3$, $X_4$, $X_5$ oder R einen basischen Rest umfaßt, dadurch gekennzeichnet, daß man eine zur Familie der Benzyl- und α-Hydroxybenzylphenyl-oside der Formel

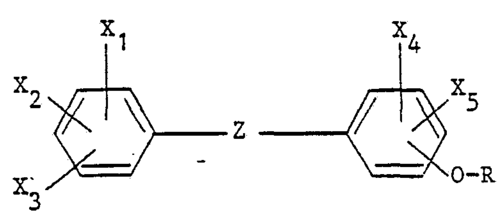

(II)

(worin Z für CO oder CHOH steht ; $X_1$, $X_2$, $X_3$, $X_4$, $X_5$ und R wie oben definiert sind) gehörende Verbindung mit einem Reduktionsmittel, ausgewählt aus $NaBH_4$ und $KBH_4$, in Trifluoressigsäure reduziert, und daß man gegebenenfalls die so erhaltene Verbindung in ein Säureadditionssalz überführt, wenn zumindest eine der Gruppen $X_1$, $X_2$, $X_3$, $X_4$, $X_5$ oder R einen basischen Rest umfaßt.

2. Verfahren nach Anspruch 1, dadurch gekennzeichnet, daß man nacheinander

(i) in eine die Verbindung II und Trifluoressigsäure enthaltende Mischung das Reduktionsmittel bei einer Temperatur von weniger oder gleich 0 °C während wenigstens 0,5 h einbrigt, wobei das Reduktionsmittel, bezogen auf die Verbindung II, im Überschuß ist ; und

(ii) sobald die Zugabe beendet ist, unter Rühren bei einer Temperatur zwischen 0 und 20 °C während 0,5 bis 12 h reagieren läßt.

3. Verwendung von Osidderivaten, ausgewählt aus der Gruppe bestehend aus :

(i) den Benzyl-phenyl-osiden der allgemeinen Formel

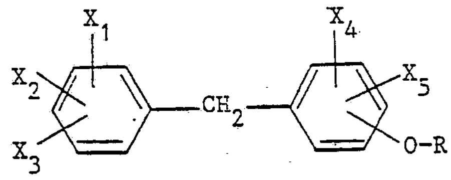

(I)

worin

$X_1$, $X_2$, $X_3$, $X_4$ und $X_5$, gleich oder verschieden, jeweils ein Wasserstoffatom, ein Halogenatom, eine $C_1$-$C_4$-Alkylgruppe, eine durch ein oder mehrere Halogenatome (insbesondere eine $CF_3$-Gruppe) substituierte $C_1$-$C_4$-Alkylgruppe, eine OH-Gruppe, eine $C_1$-$C_4$-Alkoxygruppe, eine durch ein oder mehrere Halogenatome substituierte $C_1$-$C_4$-Alkoxygruppe, eine Nitrogruppe, eine Gruppe NR'R" (worin R' und R", gleich oder verschieden, jeweils ein Wasserstoffatom, eine $C_1$-$C_4$-Alkylgruppe oder eine Acetylgruppe bedeuten), eine Methylthiogruppe, eine Methylsulfinylgruppe oder eine Mesylgruppe [—$SO_2CH_3$] darstellen,

R einen Oserest darstellt, ausgewählt aus der Gruppe bestehend aus

a) α-L-Rhamnosylrest,

b) nicht hydrolysierbaren Monosaccharidresten, und

c) nicht hydrolysierbaren Monosaccharidresten, worin die Hydroxylfunktion des Kohlenstoffatoms in Position 2 durch eine Aminfunktion ersetzt ist,

wobei die Hydroxyl- und Aminfunktionen der Gruppe R acetyliert werden können ; und

(ii) ihren Säureadditionssalzen, wenn mindestens eine der Gruppen $X_1$, $X_2$, $X_3$, $X_4$, $X_5$ oder R einen basischen Rest umfaßt, zur Herstellung einer therapeutischen Zusammensetzung mit insbesondere hypocholresterinämischer oder hypolipidämischer Wirkung.

4. Verwendung der im obigen Anspruch 3 definierten Verbindungen zur Herstellung eines hypocholesterinämischen und hypolipidämischen Medikaments zur therapeutischen Verwendung bei vermehrtem Lipidgehalt.

5. Verwendung von [4(4-Nitrobenzyl)-phenyl]-β-D-xylopyranosid zur Herstellung eines antithrombotischen Medikaments.